# FASCICULE DE BREVET EUROPEEN

(11) **EP 3 442 646 B1**
(45) Date de publication et mention de la délivrance du brevet: **29.04.2020**
(21) Numéro de dépôt: 17708840.8
(22) Date de dépôt: 09.03.2017
(51) Int. Cl.: A61M 39/22

(54) **MÉCANISME DE BLOCAGE DE RAMPE DE ROBINETS**
BLOCKIERMECHANISMUS FÜR EINEN ABSPERRHAHNKRÜMMER
BLOCKING MECHANISM FOR A STOPCOCK MANIFOLD

(30) Priorité: 15.04.2016 EP 16165515; 10.06.2016 BE 201605436
(43) Date de publication de la demande: 20.02.2019
(73) Titulaire: Trasis S.A., 4430 Ans (BE)
(72) Inventeur: TALBOT, Ludovic, 4821 Andrimont (BE); BAPLUE, Frédéric, 4557 Fraiture (Tinlot) (BE); MORELLE, Jean-Luc, 4000 Liège (BE)
(74) Mandataire: Pronovem
(86) Numéro de dépôt international: PCT/EP2017/055555
(87) Numéro de publication internationale: WO 2017/178156

(56) Documents cités:
- EP-A1- 0 637 712
- EP-A1- 1 602 388
- US-A- 3 477 469
- US-A- 4 432 392
- US-A- 4 447 236
- US-A- 6 099 511

## Description

### Objet de l'invention

La présente invention se rapporte à un mécanisme de maintien ou de blocage d'une rampe de robinets ou de robinets individuels sur des accouplements d'actionneurs mécanisés, par exemple rotatifs, automatisés ou non.

### État de la technique

### Définitions

On appelle rampe de robinet(s) (en anglais : « stopcock valve manifold ») un composant, réalisé en matière quelconque, constitué d'un *corps* de rampe muni de *raccords* d'entrée/sortie de fluide, liquide ou gaz, dans lequel sont inclus un ou plusieurs *robinets.* Ce type de composant est souvent conçu pour être à usage unique.

Il s'agit notamment d'un composant permettant, moyennant un actionnement manuel ou automatisé du (des) robinet(s), de gérer des flux de liquides et/ou de gaz dans le cadre de manipulations généralement associées aux domaines pharmaceutique et médical.

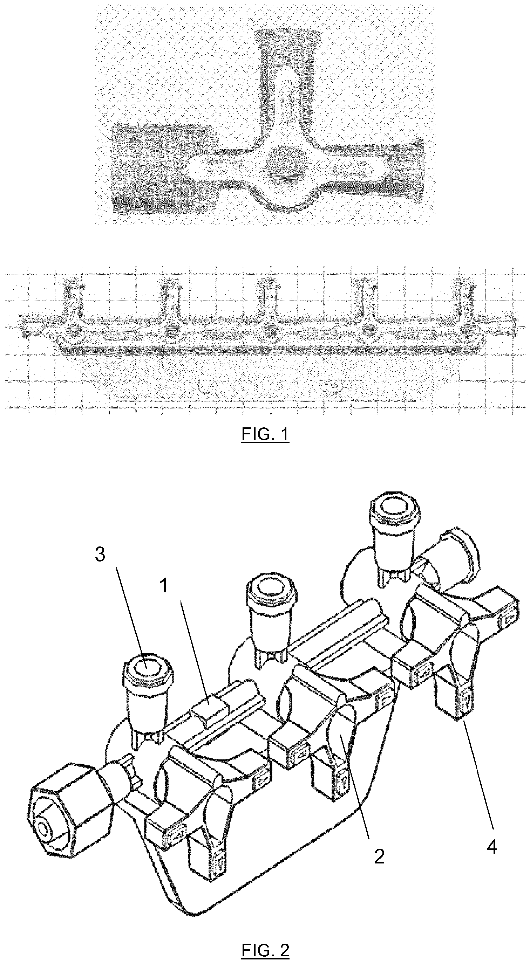

Des exemples sont montrés sur les figures 1 et 2. En particulier la figure 2 montre un corps de rampe de robinets 1 avec des raccords d'entrée/sortie 3, sur lequel sont montés trois robinets 2, ayant chacun au moins un doigt de robinet ou maneton 4 (ici en l'occurrence trois). Voir par exemple www.nordsonmedical.com, US 2011/0011474 A1, etc.

### Arrière-plan technologique et problème à résoudre

Des dispositifs d'actionnement mécanisé, automatisés ou non, des robinets de rampes de robinets ont été mis sur le marché dans divers domaines d'application, notamment en chimie radio-pharmaceutique afin de :
- protéger l'opérateur d'éventuels dangers, par exemple lorsque certains fluides sont radioactifs ;
- faciliter la tâche de l'opérateur, les robinets étant actionnées par des moteurs ou des vérins ;
- sécuriser des procédés qui peuvent inclure des séquences d'actionnement complexes.

Quel que soit le champ d'application, le principe consiste à accoupler les robinets avec des *actionneurs rotatifs,* par l'intermédiaire de pièces d'accouplement adaptées à la forme des manetons des robinets qui servent à transmettre le mouvement de rotation au robinet.

Que la rampe de robinet soit placée horizontalement ou verticalement, elle ne tient pas spontanément en place dans les accouplements à cause des forces de gravité et des mouvements transmis aux robinets par les accouplements lors de leur actionnement. Il convient donc de la maintenir en place au moyen de l'un ou l'autre dispositif adéquat.

### Art antérieur

Les rampes de robinets sont de diverses origines commerciales et ont des formes et dimensions variables. On ne connaît donc pas de mécanisme de fixation/blocage universel.

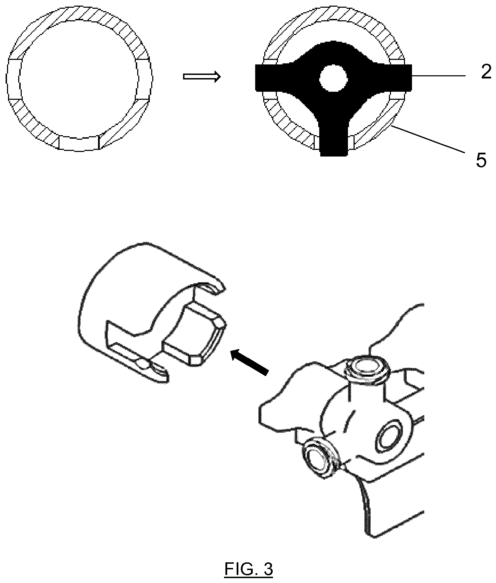

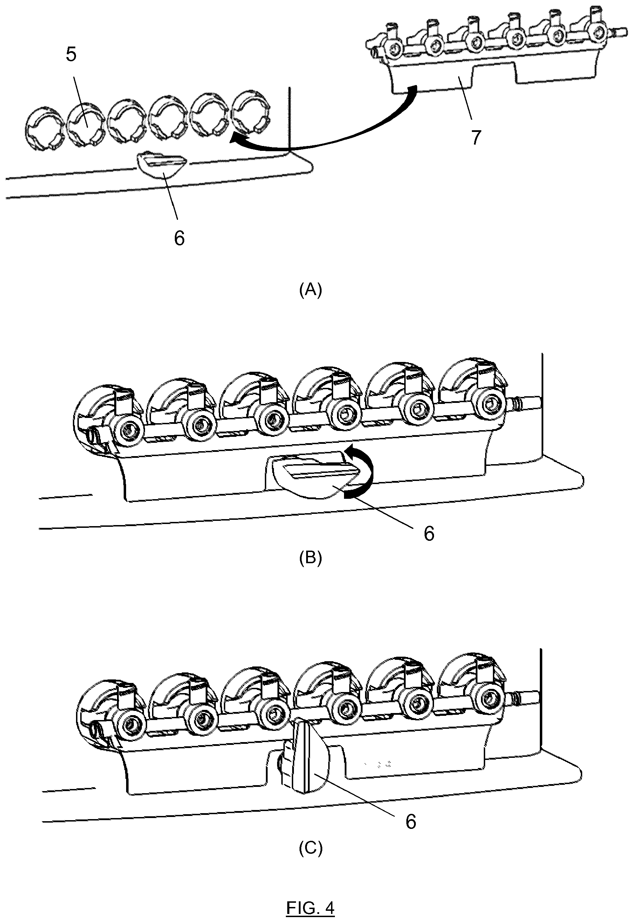

Comme représenté sur les figures 3 et 4, une solution usuellement adoptée consiste à insérer les robinets 2 dans des accouplements rotatifs à encoches ouvertes 5 (avec ou sans centrage) et à maintenir la rampe de robinets 7 dans les encoches au moyen d'un *loquet* 6 actionné manuellement ou automatiquement et qui maintient la rampe dans son ensemble. La figure 4 montre respectivement les étapes d'insertion (A) de la rampe de robinets 7 dans les accouplements à encoches ouvertes 5, d'actionnement du loquet 6 (B) et d'obtention de la position bloquée de la rampe (C).

L'opérateur doit donc effectuer le placement de la rampe de robinets en deux étapes:
- insérer la rampe de robinets dans les accouplements d'actionneurs et l'y maintenir ;
- actionner le mécanisme de maintien ou blocage.

Ainsi, les robinets ne sont pas directement maintenus par les accouplements eux-mêmes, mais par le truchement de pièces de maintien de l'ensemble de la rampe, souvent encombrantes et ne garantissant pas toujours de manière absolue l'insertion de chaque maneton de robinet dans son accouplement.

Pour ce qui concerne les rampes de robinets montées sur les appareils commercialisés dans le domaine pharmaceutique et médical, il y a souvent des appuis situés à l'avant de la rampe de robinets, tels que des molettes, permettant un blocage dans la direction de l'axe des robinets. Le mécanisme de maintien est alors souvent constitué d'une plaque mobile qui peut soit se déplacer selon l'axe des robinets, soit être escamotable par pivotement.

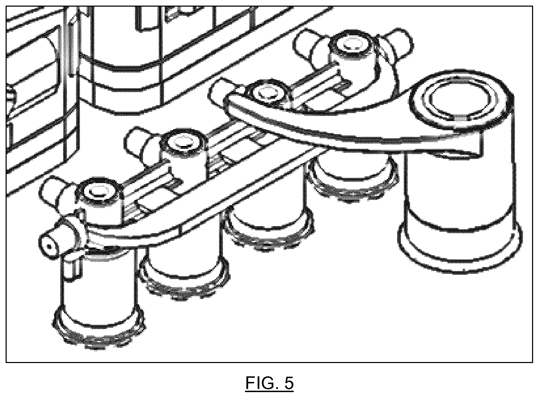

Un autre exemple de mécanisme de maintien, représenté sur la figure 5, est monté sur appareil commercialisé par la demanderesse (TRASIS UniDose™).

### Buts de l'invention

La présente invention vise à fournir un dispositif qui permette de s'affranchir des inconvénients de l'état de la technique.

En particulier, l'invention a pour but d'assurer un placement aisé d'une rampe de robinets et de permettre le maintien ou le blocage de cette rampe de manière fiable en position d'utilisation.

L'invention vise encore à proposer une solution originale pour maintenir une rampe de robinets en place, directement intégrée aux accouplements eux-mêmes.

L'invention vise encore à fournir un mécanisme où chaque accouplement maintient son propre robinet.

### Principaux éléments caractéristiques de l'invention

La présente invention se rapporte à un système de rampe de robinets, comprenant une rampe munie d'un ou plusieurs robinets, ayant chacun un moyen de contrôle rotatif d'ouverture/fermeture comprenant au moins un doigt ou maneton, d'une pièce d'accouplement d'actionneur mécanisé rotatif pour chaque robinet, l'axe rotatif de ladite pièce d'accouplement correspondant en utilisation à l'axe de rotation dudit moyen de contrôle, l'accouplement avec le robinet étant produit par insertion de chaque maneton dans une encoche réalisée dans ladite pièce d'accouplement et d'un système de maintien conçu pour que chaque pièce d'accouplement maintienne en place individuellement le robinet correspondant, caractérisé en ce que :
- la pièce d'accouplement comporte une encoche fermée, apte à recevoir un des manetons du robinet correspondant, de sorte que ce maneton puisse s'insérer dans la pièce d'accouplement correspondante en inclinant la rampe (vers l'avant) et en la redressant ensuite, jusqu'à butée sur un premier appui, dit appui avant, se trouvant au niveau de l'encoche fermée ;
- un loquet permet de maintenir la rampe dans son ensemble dans cette position redressée, par pression d'une extrémité dudit loquet sur la rampe, définissant un second appui, dit appui arrière.

On notera que, dans la configuration de l'invention, la position normale d'utilisation de la rampe de robinets est la position verticale, c'est-à-dire correspondant à un écoulement vertical. La position « avant » de la rampe désigne une position proximale de l'appareil et la position « arrière » de la rampe désigne une position distale de l'appareil.

Ainsi, selon l'invention, une des encoches ouvertes des pièces d'accouplement, communément utilisées dans l'état de la technique, est remplacée par une encoche fermée. Selon l'invention, une fois le maneton introduit obliquement dans cette encoche fermée, le bord de l'encoche va faire office de butée et va empêcher, à proximité de l'appareil, tout mouvement de retour du maneton en s'éloignant de l'appareil (butée « avant »). L'autre point d'appui sera constitué par l'extrémité du loquet qui pousse sur une partie arrière de la rampe (butée « arrière »), ce qui empêche, à distance de l'appareil, tout mouvement de la rampe en se rapprochant de l'appareil. Comme les deux points d'appui se trouvent à des positions distinctes et opposées, aucun couple ne peut plus faire basculer/pivoter la rampe rendant, en l'état, son retrait impossible.

Selon des formes d'exécution préférées de l'invention, le système de rampe de robinets comporte en outre une des caractéristiques suivantes, ou encore une combinaison appropriée de plusieurs d'entre elles :
- le loquet est un loquet passif, du type à ressort ;
   le loquet à ressort est une languette conçue pour s'écarter de son point d'équilibre lorsque la rampe, en s'insérant obliquement dans les encoches fermées des pièces d'accouplement, vient pousser simultanément sur l'extrémité précitée du loquet et pour revenir à son point d'équilibre une fois que la rampe a été redressée, tout en bloquant la rampe par butée au niveau de son appui arrière ;
- le loquet est un loquet actif ou automatisé ;
   le loquet comporte un détecteur de position de la rampe et un actionneur déclenchant un poussoir lorsque le détecteur détecte la position redressée de la rampe.

### Brève description des figures

Les figures 1 et 2 montrent des exemples de rampe de robinets selon l'état de la technique.
La figure 3 montre le principe général d'un accouplement à encoches ouvertes, selon l'état de la technique.
La figure 4 montre les différentes étapes de l'accouplement à encoches ouvertes.
La figure 5 montre un exemple de mécanisme de maintien d'une rampe de robinets selon l'état de la technique, au moyen d'une pièce de maintien de l'ensemble de la rampe.
La figure 6 montre le principe général d'un accouplement à encoche fermée, selon une forme d'exécution de la présente invention.
La figure 7 montre les différentes étapes de l'accouplement à encoche fermée et lame ressort, selon une forme d'exécution de la présente invention.

### Description d'une forme d'exécution préférée de l'invention

L'invention consiste en la mise en œuvre d'un principe de maintien des rampes de robinets sur le mécanisme d'actionnement dans lequel les robinets sont individuellement maintenus dans leur accouplement. La mise en place de la rampe de robinets s'effectue en un seul et simple mouvement.

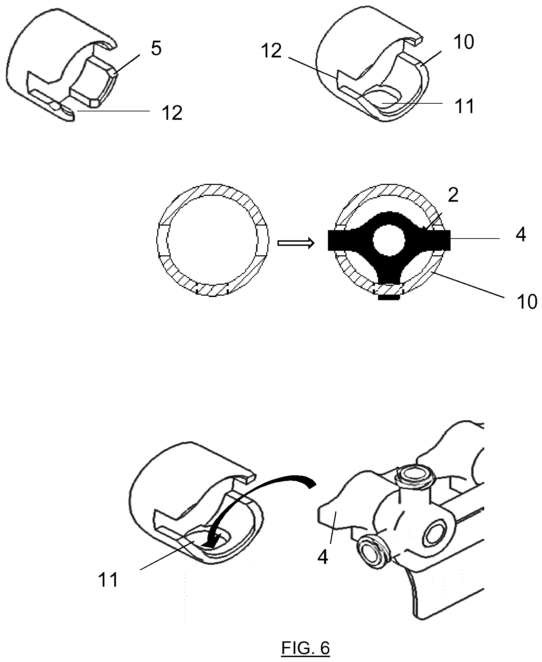

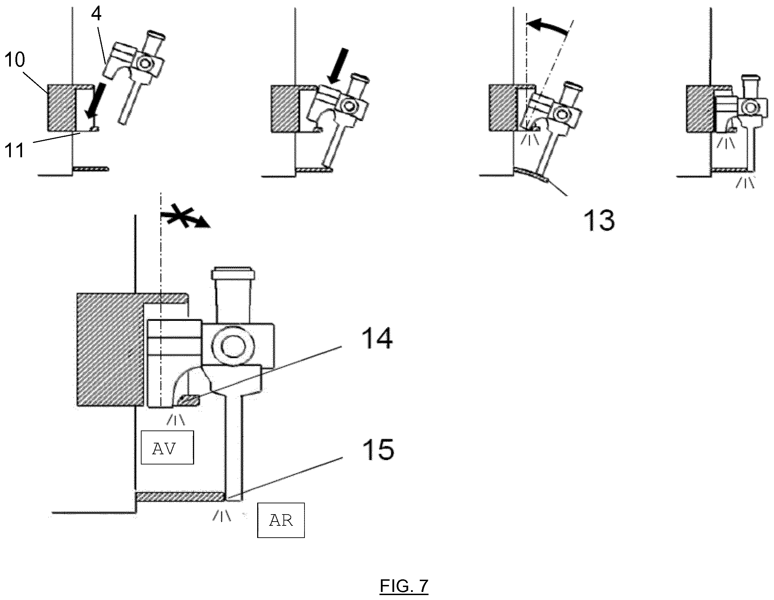

Le dispositif consiste en la combinaison d'éléments suivante (figures 6 et 7) :
- l'une des encoches ouvertes 12 de la pièce d'accouplement 10 est remplacée par un trou fermé 11. Lors de la mise en place de la rampe, un des doigts 4 de chaque robinet 2 s'insère dans ce trou 11. La rampe est approchée obliquement pour permettre l'insertion. Une fois le robinet 2 inséré dans l'accouplement 10, la rampe est basculée de telle sorte que les axes de rotation du robinet et de l'actionneur soient alignés. Chaque robinet 2 est alors maintenu dans son accouplement 10, avec un appui 14 à l'avant de la rampe, au niveau du bord du trou fermé 11 ;
- un loquet 13 par exemple sur ressort ou automatisé vient alors s'appuyer à l'arrière 15 du corps de la rampe de robinets, ce qui empêche alors la rampe d'effectuer un mouvement de rotation qui lui permettrait de sortir des accouplements 10. La rampe est donc strictement maintenue en place dans les actionneurs par le truchement de deux appuis simples, situés respectivement à l'avant 14 et à l'arrière 15 de l'appareil ou la rampe de robinets.

### Avantages de l'invention

L'invention procure notamment les avantages suivants :
- mise en place en un tour de main selon un mouvement unique, simple et naturel ;
- mécanisme de blocage simplifié, qu'il soit passif (sur ressort) ou actif (actionné automatiquement) :
   - dispositif de loquet plus facile à manipuler ;
   - dispositif de loquet facile à automatiser ;
   - pas de risque d'oubli d'actionnement.

### Repères de référence

- 1: Corps de rampe de robinets
- 2: Robinet
- 3: Raccord d'entrée/sortie
- 4: Doigt de robinet ou maneton
- 5: Accouplement d'actionneur rotatif à encoche(s) ouverte(s)
- 6: Loquet de blocage de rampe
- 7: Rampe de robinets
- 10: Accouplement d'actionneur rotatif à encoche fermée
- 11: Encoche fermée ou « trou »
- 12: Encoche ouverte
- 13: Loquet passif (ressort) ou actif (automatisé)
- 14: Appui avant
- 15: Appui arrière

## Revendications

1. Système de rampe de robinets comprenant une rampe (7) munie d'un ou plusieurs robinets (2), ayant chacun un moyen de contrôle rotatif d'ouverture/fermeture comprenant au moins un doigt ou maneton (4), d'une pièce d'accouplement d'actionneur mécanisé rotatif (5, 10) pour chaque robinet (2), l'axe rotatif de ladite pièce d'accouplement correspondant en utilisation à l'axe de rotation dudit moyen de contrôle, l'accouplement avec le robinet (2) étant produit par insertion de chaque maneton (4) dans une encoche (11, 12) réalisée dans ladite pièce d'accouplement (5, 10) et d'un système de maintien conçu pour que chaque pièce d'accouplement (5, 10) maintienne en place individuellement le robinet (2) correspondant, **caractérisé en ce que** :
- la pièce d'accouplement (10) comporte une encoche fermée (11), apte à recevoir un des manetons (4) du robinet correspondant (2), de sorte que ce maneton (4) puisse s'insérer dans la pièce d'accouplement (10) correspondante en inclinant la rampe (7) et en la redressant ensuite, jusqu'à butée sur un premier appui, dit appui avant (14), constitué par le bord de l'encoche fermée (11) ;
- un loquet (13) permet de maintenir la rampe (7) dans son ensemble dans cette position redressée, par pression d'une extrémité dudit loquet (13) sur la rampe (7), définissant un second appui, dit appui arrière (15).

2. Système de rampe de robinets selon la revendication 1, **caractérisé en ce que** le loquet (13) est un loquet passif, du type à ressort.

3. Système de rampe de robinets selon la revendication 2, **caractérisé en ce que** le loquet à ressort (13) comporte une languette conçue pour s'écarter de son point d'équilibre lorsque la rampe (7), en s'insérant obliquement dans les encoches fermées (11) des pièces d'accouplement (10) vient pousser simultanément sur l'extrémité précitée du loquet (13) et pour revenir à son point d'équilibre une fois que la rampe (7) a été redressée, tout en bloquant la rampe (7) par butée au niveau de son appui arrière (15).

4. Système de rampe de robinets selon la revendication 1, **caractérisé en ce que** le loquet (13) est un loquet actif ou automatisé.

5. Système de rampe de robinets selon la revendication 4, **caractérisé en ce que** le loquet (13) comporte un détecteur de position de la rampe et un actionneur déclenchant un poussoir lorsque le détecteur détecte la position redressée de la rampe.

## Patentansprüche

1. Absperrhahnkrümmersystem, umfassend einen Krümmer (7), ausgestattet mit einem oder mehreren Absperrhähnen (2), von denen jeder ein rotierendes öffnendes/schließendes Steuermittel hat, umfassend mindestens einen Finger oder Zapfen (4), einem Kopplungsteil eines rotierenden mechanisierten Aktuators (5, 10) für jeden Absperrhahn (2), wobei die Rotationsachse des Kopplungsteils in Verwendung der Rotationsachs des Steuermittels entspricht, wobei die Kopplung mit dem Absperrhahn (2) durch Eingreifen jedes Zapfens (4) in eine Aussparung (11, 12) erfolgt, die im Kopplungsteil (5, 10) eingearbeitet ist und einem Haltesystem, das ausgebildet ist, damit jedes Kopplungsteil (5, 10) den entsprechenden Absperrhahn (2) individuell am Platz hält, **dadurch gekennzeichnet, dass**:
- das Kopplungsteil (10) eine geschlossene Aussparung (11) aufweist, die imstande ist, einen der Zapfen (4) des entsprechenden Absperrhahns (2) aufzunehmen, derart, dass dieser Zapfen (4) in das entsprechende Kopplungsteil (10) durch Neigen des Krümmers (7) und darauffolgendes Aufrichten desselben eingreifen kann, bis zum Anschlag an einer ersten Stütze, bezeichnet als vordere Stütze (14), die aus dem Rand der geschlossenen Aussparung (11) besteht;
- ein Riegel (13) erlaubt, den Krümmer (7) in seiner Gesamtheit in dieser aufgerichteten Position zu halten, durch Druck eines Endes des Riegels (13) auf den Krümmer (7), das eine zweite Stütze, bezeichnet als hintere Stütze (15), definiert.

2. Absperrhahnkrümmersystem nach Anspruch 1, **dadurch gekennzeichnet, dass** der Riegel (13) ein passiver Riegel vom Typ mit Feder ist.

3. Absperrhahnkrümmersystem nach Anspruch 2, **dadurch gekennzeichnet, dass** der Federriegel (13) eine Zunge aufweist, die ausgebildet ist, um sich von ihrem Gleichgewichtspunkt zu beabstanden, wenn der Krümmer (7), wenn er schräg in die geschlossenen Aussparungen (11) der Kopplungsteile (10) eingreift, gleichzeitig auf das vorgenannte Ende des Riegels (13) drückt und um zu seinem Gleichgewichtspunkt zurückzukehren, sobald der Krümmer (7) aufgerichtet wurde, bei Blockade des Krümmers (7) durch Anschlag im Bereich seiner hinteren Stütze (15).

4. Absperrhahnkrümmersystem nach Anspruch 1, **dadurch gekennzeichnet, dass** der Riegel (13) ein aktiver oder automatisierter Riegel ist.

5. Absperrhahnkrümmersystem nach Anspruch 4, **dadurch gekennzeichnet, dass** der Riegel (13) einen Positionsdetektor des Krümmers und einen Aktuator aufweist, der einen Drücker auslöst, wenn der Detektor die aufgerichtete Position des Krümmers feststellt.

## Claims

1. A tap ramp system comprising a ramp (7) provided with one or several taps (2), each having a rotary opening/closing control means comprising at least one finger or crank pin (4), a mechanized rotary actuator coupling part (5, 10) for each tap (2), the rotation axis of said coupling part corresponding during use to the rotation axis of said control means, the coupling with the tap (2) being achieved by inserting each crank pin (4) into a notch (11, 12) made in said coupling part (5, 10) and a maintaining system designed so that each coupling part (5, 10) individually keeps the corresponding tap (2) in place, **characterized in that**:
- the coupling part (10) comprises a closed notch (11), designed to receive one of the crank pins (4) of the corresponding tap (2), such that this crank pin (4) can be inserted in the corresponding coupling part (10) by inclining the ramp (7) and next straightening it, until it abuts on a first support, called front support (14), formed by the edge of the closed notch (11);
- a latch (13) allows to keep the ramp (7) as a whole in this straightened position, by pressing one end of said latch (13) on the ramp (7), defining a second support, called rear support (15).

2. The tap ramp system according to Claim 1, **characterized in that** the latch (13) is a passive latch, of the spring type.

3. The tap ramp system according to Claim 2, **characterized in that** the spring latch (13) comprises a tongue designed to move away from its equilibrium point when the ramp (7), by being inserted obliquely into the closed notches (11) of the coupling parts (10), simultaneously pushes on the aforementioned end of the latch (13) and returns to its equilibrium point once the ramp (7) has been straightened, while blocking the ramp (7) by abutting at the level of its rear support (15).

4. The tap ramp system according to Claim 1, **characterized in that** the latch (13) is an active or automated latch.

5. The tap ramp system according to Claim 4, **characterized in that** the latch (13) comprises a ramp position detector and an actuator triggering a push-piece when the detector detects the straightened position of the ramp.
